(19) Europäisches Patentamt
European Patent Office
Office européen des brevets

(11) Veröffentlichungsnummer: 0 071 166
A1

# (12) EUROPÄISCHE PATENTANMELDUNG

(21) Anmeldenummer: 82106563.8

(22) Anmeldetag: 21.07.82

(51) Int. Cl.$^3$: C 07 C 45/36
C 07 C 47/54, C 07 C 47/542
C 07 C 47/575, C 07 C 47/55

(30) Priorität: 24.07.81 DE 3129194

(43) Veröffentlichungstag der Anmeldung:
09.02.83 Patentblatt 83/6

(84) Benannte Vertragsstaaten:
BE CH DE FR GB IT LI NL

(71) Anmelder: HOECHST AKTIENGESELLSCHAFT
Postfach 80 03 20
D-6230 Frankfurt/Main 80(DE)

(72) Erfinder: Kuckertz, Herbert, Dr.
Ludwig-Schäfer-Weg 11
D-6233 Kelkheim (Taunus)(DE)

(72) Erfinder: Schaeffer, Georg, Dr.
Herderstrasse 58
D-6238 Hofheim am Taunus(DE)

## (54) Verfahren zur Herstellung von aromatischen Aldehyden.

(57) Aromatische Aldehyde werden hergestellt durch Oxidation von Methylaromaten mit einem molekularen Sauerstoff enthaltenden Gas - vorzugsweise Luft -
in Fremlösemittel-freier flüssiger Phase
in Gegenwart eines Kobaltkatalysators und gegebenenfalls von Brom und/oder Bromverbindungen
bei erhöhter Temperatur
im Einstufenverfahren
unter ständiger Abführung des bei der Reaktion gebildeten Wassers.

Das Verfahren liefert hohe Aldehyd-Selektivitäten bei nicht zu niedrigen Umsätzen.

EP 0 071 166 A1

Verfahren zur Herstellung von aromatischen Aldehyden

Aromatische Aldehyde wie z.B. Benzaldehyd, p-tert.-Butylbenzaldehyd, Anisaldehyd, 3-Phenoxybenzaldehyd etc. sind hauptsächlich Zwischenprodukte auf verschiedenen Sachgebieten wie dem Gebiet der Geschmacks- und Geruchsstoffe, dem Farbstoff-, Pflanzenschutz- und Pharmasektor etc.

Zur Herstellung aromatischer Aldehyde ist eine Reihe verschiedener Methoden bekannt. Einige dieser Methoden bestehen in der Oxidation von methylsubstituierten aromatischen Verbindungen (Methylaromaten). Dabei muß bei besonders sorgfältig ausgewählten Bedingungen gearbeitet werden, weil die Weiteroxidation der Aldehyd- und Carboxylgruppe im allgemeinen schneller verläuft als die Oxidation der Methylgruppe in der Ausgangsverbindung zur Aldehydgruppe.

Bevorzugte Oxidationsmittel bei diesen Methoden sind molekularen Sauerstoff enthaltende Gase, insbesondere Luft. Die Oxidation kann hier sowohl in der Gas- als auch in flüssiger Phase durchgeführt werden. Bei den Flüssigphasen-Methoden kann man wiederum unterscheiden zwischen solchen, die in einem Fremdlösemittel arbeiten, und solchen bei denen keine Fremdlösemittel zugegen ist.

Bevorzugte Lösemittel bei den in Fremdlösemitteln arbeitenden Oxidationsverfahren sind niedere gesättigte aliphatische Carbonsäuren - insbesondere Essigsäure - und/oder deren Anhydride. Ein solches Oxidationsverfahren ist z.B. beschrieben in der DE-OS 27 57 031. Danach werden Toluolderivate mit einer oder mit zwei Etherbindungen - also z.B. o-, m- und p-Methoxytoluol, die Phenoxytoluole etc. - mit einem molekularen Sauerstoff enthaltenden Gas, unter Verwendung einer niederen aliphatischen gesättigten Carbonsäure - vorzugsweise Essigsäure - und/oder eines Anhydrids einer solchen Säure als Lösemittel in Gegenwart eines löslichen Kobaltsalzes und einer Bromionen liefernden Substanz bei einer Temperatur im Bereich von 30 bis 200°C im Einstufenverfahren bei Umsätzen bis höchstens 90 % der Ausgangsverbindung oxidiert, wobei hauptsächlich entsprechende Aldehyd-/Alkohol-Gemische entstehen.

Die Aldehyd-Selektivität ist hierbei im allgemeinen umso höher, je niedriger der Umsatz an Ausgangsverbindung und umgekehrt (niedrigere Aldehyd-Selektivität bei höheren Umsätzen). Im Falle beispielsweise des o-Methoxytoluols als Ausgangsverbindung soll nach den Angaben der DE-OS (S. 18/19) bei Umsätzen von 70 %, 50 %, 30 % und 20 % die entsprechende Aldehyd-Selektivität 58 %, 62 %, 73 % bzw. 75 % betragen.

Das Verfahren liefert somit recht brauchbare Aldehyd-Selektivitäten und -Ausbeuten. Nachteilig ist hier jedoch die bei der Aufarbeitung des Oxidationsansatzes notwendige Abtrennung und Wiedergewinnung des Lösemittels. Im Falle der Verwendung von niederen gesättigten aliphatischen Carbonsäuren als Lösemittel fallen diese in mit Wasser (aus der Oxidationsreaktion) verdünnter Form an. Bei der Ansatz-Aufarbeitung wird dann im allgemeinen zunächst die wasserhaltige Carbonsäure abdestilliert; in einer weiteren Destillationsstufe erfolgt darauf die Wiedergewinnung der wasserfreien bzw. nur mehr wenig Wasser enthaltenden Carbonsäure, was einen nicht unerheblichen Aufwand und eine beträchtliche Verfahrensverteuerung bedeutet.

Im Falle des Einsatzes von Cabonsäureanhydriden als Lösemittel werden diese durch das Reaktionswasser in die entsprechenden aliphatischen Carbonsäuren überführt, so daß es unter Verbrauch von relativ teurem Anhydrid zu einem Zwangsanfall an erheblich billigerer Säure kommt, die - zusammen

mit dem noch vorhandenen Anhydrid - ihrerseits dann von den übrigen Komponenten des Oxidationsansatzes abgetrennt werden muß.

Der mit der Aufarbeitung lösemittelhaltiger Oxidationsansätze verbundene Aufwand und die dadurch bedingten Nachteile entfallen bei den Verfahren, welche in Abwesenheit von Fremdlösemitteln arbeiten. Ein solches Verfahren ist z.B. bekannt aus der DE-PS 1 224 291. Nach dem in dieser PS beschriebenen Verfahren wird Toluol mit Luft
in flüssiger, fremdlösemittelfreier Phase
in Abwesenheit von Oxidationskatalysatoren
bei Temperaturen in der Größenordnung von 170 bis 220°C
unter einem solchen Druck, daß das Medium flüssig bleibt,
oxidiert,
wobei die Oxidation nach einem Toluol-Umsatz von höchstens 10 %, vorzugsweise 4 bis 7 %, abgebrochen wird.

Man erhält so ein Gemisch von Toluol, Benzylhydroperoxid, Benzylalkohol, Benzaldehyd, Benzoesäure und einigen anderen Nebenprodukten, das dann z.B. durch katalytische Hydrierung oder durch Erhitzen auf 180 - 200°C unter einer Inertgasatmosphäre entperoxidiert wird. Das Verfahren liefert Benzaldehyd-Benzylalkohol-Gemische in Selektivitäten zwischen etwa 50 und 70 %, wobei Benzylalkohol den überwiegenden Gemischbestandteil ausmacht. Die PS spricht zwar von einer 50 - 70 %igen Ausbeute (nicht Selektivität), doch kann hier wohl nur Selektivität (= auf den Umsatz bezogene Ausbeute) gemeint sein; denn bei einem nur höchsten 10 %igen Umsatz kann es keine 50 - 70 %ige Ausbeute geben.

Gemische mit einem überwiegenden Aldehydanteil werden erhalten durch Zersetzung des organischen Hydroperoxids in Gegenwart einer Chromverbindung sowie einer organischen Base mit mindestens einem sekundären oder tertiären N-Atom nach dem Verfahren der DE-PS 1 913 123.

Dem Fremdlösemittel-freien Verfahren gemäß DE-PS 1 224 291 und gegebenenfalls DE-PS 1 913 123 haftet zwar der durch die relativ aufwendige Aufarbeitung fremdlösmittelhaltiger

Oxidationsansätze bedingte Nachteil nicht an, doch ist für dieses Verfahren insbesondere die 2-Stufigkeit (erste Stufe: Oxidation zum Hydroperoxid-haltigen Gemisch, zweite Stufe: Zersetzung des Hydroperoxids) nachteilig.

Es ist jedoch auch bekannt, Methylaromaten mit einem molakularen Sauerstoff enthaltenden Gas in fremdlösemittelfreier flüssiger Phase im Einstufenverfahren zu aromatischen Aldehyden zu oxidieren.

So beschreibt die JP-AS Sho-54-41578 ein Verfahren zur Oxidation von Toluol mit einem molekularen Sauerstoff enthaltenden Gas
in flüssiger Phase
in Gegenwart eines Kobalthalogenids als Katalysator (in einer Menge von 500 bis 5000 ppm - als Metall berechnet - bezogen auf Toluol)
bei Temperaturen vorzugsweise zwischen 160 und 195°C
und einem Druck zwischen 10 und 30 atü
im Einstufenverfahren;
kennzeichnendes Merkmal dieses Verfahrens ist die Anwesenheit von 4 bis 15 Vol.-% Wasser in der flüssigen Phase.

Nach den Beispielen dieser AS beträgt die Selektivität des Verfahrens bezüglich Benzaldehyd jedoch nur maximal 22 % bei einem Toluol-Umsatz von 13 %, was für eine Aldehyd-Herstellung insbesondere in technischem Maßstab kaum ausreichend ist.

Wegen des steigenden Bedarfs an aromatischen Aldehyden sowie wegen der verschiedenen Mängel der bekannten Verfahren zur Herstellung aromatischer Aldehyde durch Oxidation von Methylaromaten mit einem Sauerstoff enthaltenden Gas bestand nun die Aufgabe, die bekannten Verfahren zu verbessern oder ein neues verbessertes Verfahren zu entwickeln.

Diese Aufgabe konnte erfindungsgemäß dadurch gelöst werden, daß das bei der Oxidation von Methylaromaten mit einem molekularen Sauerstoff enthaltenden Gas in Fremdlösemittelfreier flüssiger Phase entstehende (Reaktions-)Wasser ständig aus der Reaktionsmischung entfernt wird.

Erfindungsgegenstand ist somit ein Verfahren zur Herstellung von aromatischen Aldehyden durch Oxidation von Methylaromaten mit einem molekularen Sauerstoff enthaltenden Gas in fremdlösemittelfreier flüssiger Phase,
in Gegenwart eines Kobalt-Katalysators und gegebenenfalls von Brom und/oder Bromverbindungen.
bei erhöhter Temperatur
im Einstufenverfahren;
das Verfahren ist dadurch gekennzeichnet, daß man die Oxidation unter ständiger Abführung des bei der Reaktion gebildeten Wassers durchführt.

Das Verfahren liefert bei Umsätzen bis zu etwa 25 % Aldehyd-Selektivitäten durchweg zwischen etwa 40 und 80 % - im Ausnahmefall auch darüber, was in der Größenordnung der relativ die höchsten Aldehyd-Selektivitäten ergebenden in Fremdlösemitteln arbeitenden entsprechenden bekannten Verfahren liegt. Gegenüber diesen in Fremdlösemitteln arbeitenden bekannten Verfahren besitzt das erfindungsgemäße Verfahren den Vorteil der einfacheren Aufarbeitung des Reaktionsansatzes (infolge der Abwesenheit von Fremdlösemitteln).

Gegenüber dem fremdlösemittelfreien 2-Stufenverfahren gemäß DE-PS 1 224 291 und gegebenenfalls DE-PS 1 913 123 zeichnet sich die Erfindung vor allem durch ihre Einstufigkeit aus.

Der Vorteil der Erfindung gegenüber dem fremdlösemittelfreien Einstufenverfahren gemäß JP-AS Sho-54-41578 ist hauptsächlich in der höheren Wirtschaftlichkeit (infolge der höheren Aldehyd-Selektivitäten) zu sehen.

Es war außerordentlich überraschend, daß durch die Abwesenheit bzw. die ständige Entfernung des Reaktionswassers bei dem fremdlösemittelfreien 1-stufigen Verfahren der Flüssigphasenoxidation von Methylaromaten eine gegenüber dem Verfahren der JP-AS Sho 54-41578 ganz beträchtlich höhere Aldehyd-Selektivität erzielt wird (Erfindung: zwischen etwa 40 und 80 % bei Umsätzen bis etwa 25 %, JP-AS Sho 5441578: maximal etwa 22 % bei 13 % Toluol-Umsatz); nach der erwähnten JP-AS mußte man nämlich annehmen, daß die fremdlösemittelfreie einstufige Flüssigphasenoxidation von Methylaromaten zu den entsprechenden Aldehyden mit einem Sauerstoff enthaltenden Gas überhaupt erst durch die Anwesenheit und Aufrechterhaltung einer bestimmten Wassermenge in einigermaßen diskutabler Weise ermöglicht wird.

Als Ausgangsverbindungen für das erfindungsgemäße Verfahren kommen im Prinzip alle möglichen bei den angewandten Temperatur- und Druckbedingungen flüssigen Methylaromaten, die noch durch unter den angewandten Reaktionsbedingungen nicht reagierende (inerte) Gruppen substituiert sein können, in Frage. Einige beispielhafte Ausgangsverbindungen sind nachstehend formelmäßig aufgeführt:

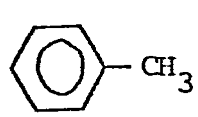
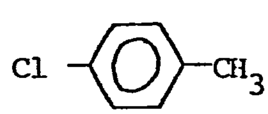
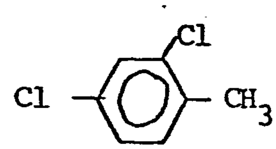

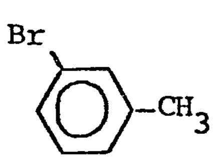
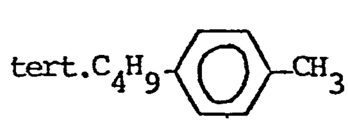
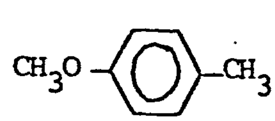

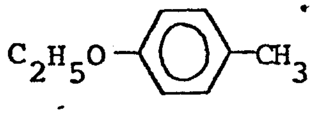

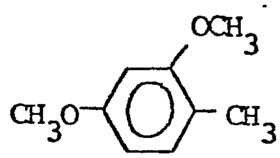

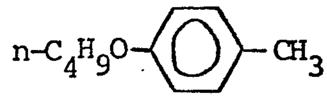

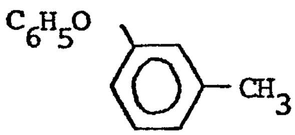

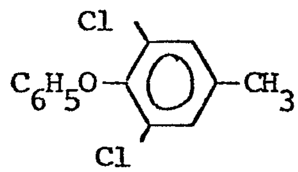

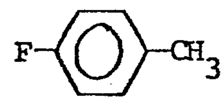

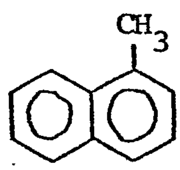

etc.

Bevorzugte Ausgangsverbindungen sind die unter die folgende Formel I fallenden Methylaromaten:

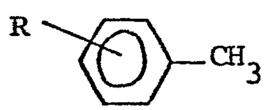

(I)

worin R = H, Halogen (vorzugsweise F, Cl,Br), tert.-Butyl, OR' (R' = Alkyl, vorzugsweise $C_1$-$C_4$-Alkyl, oder Aryl, vorzugsweise Phenyl)

Als molekularen Sauerstoff enthaltendes Gas können sowohl reiner Sauerstoff als auch Gemische von Sauerstoff mit Inertgasen verwendet werden. Bevorzugtes Oxidationsmittel ist Luft.

Als Kobalt-Katalysatoren sind im Prinzip alle für derartige Oxidationen bekannten und üblichen Kobalt-Verbindungen einsetzbar. Dies sind im allgemeinen die Kobaltsalze organischer und anorganischer Säuren, z.B. Kobalt-Acetat, -Propionat, -Benzoat, -Naphthoat, -Chlorid, -Bromid etc.

Bevorzugt ist der Einsatz von Kobaltverbindungen aromatischer

Carbonsäuren, insbesondere der bei der Oxidation der eingesetzten Methylaromaten als Nebenprodukte entstehenden aromatischen Carbonsäuren. Besonders bevorzugte Kobaltverbindungen sind also z.B. im Falle der Benzaldehyd-Herstellung Cobaltbenzoat oder im Falle der p-tert.-Butylbenzaldehyd-Herstellung Kobalt-p-tert.-butylbenzoat.

Es ist dabei nicht unbedingt erforderlich, diese besonders bevorzugten Kobalt-Verbindungen als solche dem Reaktionsansatz zuzusetzen; man kann sie etwa auch vor Beginn oder während der Reaktion im Reaktionsmedium herstellen. Eine einfache Art der Herstellung im Reaktionsmedium besteht z.B. darin, daß man das Substrat mit der jeweiligen aromatischen Carbonsäure versetzt und Kobaltcarbonat oder -acetat zugibt. Im Falle der Carbonatzugabe bildet sich das Salz der aromatischen Carbonsäure dann unter $CO_2$-Entwicklung, im Falle der Acetatzugabe unter Freisetzung von Essigsäure, die vor oder auch während der Reaktion abdestilliert werden kann.

Die Menge an Kobalt-Katalysator im Reaktionsgemisch kann innerhalb relativ weiter Grenzen - im allgemeinen zwischen etwa 0,01 und 10 Gew.-% (als Metall berechnet und auf den Ausgangs-Methylaromaten bezogen) - variieren. Die bevorzugte Kobaltkatalysator-Menge liegt zwischen etwa 0,1 und 1 Gew.-% (berechnet als Metall und bezogen auf die Ausgangsverbindung).

Der Kobalt-Katalysator kann gegebenenfalls auch durch Zusätze anderer Schwermetall-(beispielsweise Mangan)-Verbindungen und/oder von Brom ($Br_2$) und/oder Bromverbindungen modifiziert werden.

Als Bromverbindungen kommen in beispielhafter Weise in Frage: Bromwasserstoff HBr, Alkalibromide, Erdalkalibromide, Schwermetallbromide ($CoBr_2$, $MnBr_2$ etc.), Alkylbromide etc.

Die Menge des Broms und/oder der Bromverbindungen wird vorteilhaft so bemessen, daß zwischen etwa 0,05 und 5, vorzugsweise zwischen etwa 0,1 und 1 Gew.-% Brom im Reaktionsmedium vorhanden sind.

Die Reaktionstemperaturen liegen im allgemeinen zwischen etwa 50 und 250°C. Der Temperaturbereich, in dem die Reaktion vorzugsweise durchgeführt wird, hängt davon ab, ob man in Gegenwart oder in Abwesenheit von Brom und/oder Bromverbindungen arbeitet.

In Gegenwart letzterer (Brom und/oder Bromverbindungen) liegt die Reaktionstemperatur vorteilhaft etwas niedriger (vorzugsweise bei etwa 50 bis 150°C) als in deren Abwesenheit (Reaktionstemperatur vorzugsweise bei etwa 150 bis 250°C).

Der Druck, bei dem das erfindungsgemäße Verfahren durchgeführt wird, kann normalerweise zwischen etwa 0,5 und 100 bar liegen; bevorzugt ist Normaldruck.

Auf den zweckmäßig gewählten Druckbereich hat natürlich einerseits der Siedepunkt des Ausgangsmaterials und andererseits die Gegenwart oder Abwesenheit von Brom und/oder Bromverbindungen einen gewissen Einfluß. So ist z.B. im Falle der Oxidation von Toluol in Gegenwart von Brom und/oder Bromverbindungen im Temperaturbereich von etwa 80 bis 100°C (d.h. unterhalb der Siedepunkte von Toluol und Benzaldehyd) ein Arbeiten bei Normaldruck möglich und vorteilhaft. Will man dagegen Toluol in Abwesenheit von Brom und/oder Bromverbindungen zu Benzaldehyd oxidieren, so arbeitet man zweckmäßiger bei etwa 100 bis 200°C und einem Druck von etwa 1,5 bis 20 bar.

Eine weitere Möglichkeit der Beeinflussung der Höhe des zweckmäßigen Reaktionsdruckes ist der Sauerstoffgehalt des molekularen Sauerstoff enthaltenden Gases. Beim Einsatz von reinem Sauerstoff genügt ein niedrigerer Druck als beim

Einsatz etwa von Luft.

Um eine mehr als 40 %ige Selektivität an dem gewünschten Aldehyd zu erreichen, arbeitet man in Gegenwart von Brom und/oder Bromverbindungen zweckmäßig bei Umsätzen bis zu etwa 25 %, vorzugsweise zwischen etwa 5 und 15 %, in Abwesenheit von Brom und/oder Bromverbindungen vorteilhaft bei Umsätzen nur bis zu etwa 15 %, vorzugsweise zwischen etwa 5 und 10 %, der Ausgangs-Methylaromaten.

Dies bedeutet, daß in Gegenwart von Brom und/oder Bromverbindungen im allgemeinen bei etwas höheren Umsätzen gearbeitet werden kann als in Abwesenheit solcher Substanzen (bei vergleichbaren Selektivitäten). Die bromfreie Arbeitsweise zeichnet sich dafür durch Vorteile einer einfacheren Aufarbeitung und Katalysatorrückführung aus. Außerdem gestattet die bromfreie Arbeitsweise die Verwendung wesentlich preiswerterer Apparate-Werkstoffe.

Hauptsächliches Nebenprodukt der Reaktion ist die jeweilige aromatische Carbonsäure. Mit steigenden Umsätzen erhöht sich ihr Anteil immer mehr. Der Anstieg des Anteils an freier Carbonsäure im Reaktionsmedium wirkt sich im allgemeinen günstig auf die Aldehyd-Selektivität aus, so daß es sich empfiehlt, sie von vorneherein dem Ansatz zuzusetzen bzw. sie bei kontinuierlicher Arbeitsweise zu einem gewissen Teil zurückzuführen. Im Falle des Zusatzes von Brom und/oder Bromverbindungen bei der Oxidationsreaktion zählt zu den Nebenprodukten oft auch das jeweilige Benzylbromid.

Zur Vermeidung unerwünscht hoher Mengen an Nebenprodukten ist eine laufende Kontrolle des Reaktionsfortgangs z.B. durch gaschromatographische Analyse zweckmäßig.

Die Aufarbeitung des Oxidationsansatzes geschieht auf übliche Weise z.B. durch fraktionierte Destillation der gegebenenfalls von ausgefallenen Feststoffen (Carbonsäuren!) abgetrennten und u.U. noch einer Alkaliwäsche unterzogenen flüssigen Phase.

Im Falle der Durchführung einer Alkaliwäsche kann der Kobalt-Katalysator

aus der wäßrigen Phase leicht wiedergewonnen werden.

Insbesondere wegen der einfachen Durchführbarkeit und Aufarbeitung des Reaktionsansatzes sowie der hohen Aldehyd-Selektivitäten und Ausbeuten bei nicht zu niedrigen Umsätzen stellt die Erfindung einen erheblichen Fortschritt dar.

Die Erfindung wird nun durch die folgenden Beispiele näher erläutert.

Beispiel 1

Herstellung von p-tert.-Butylbenzaldehyd bei 80 - 100°C in Gegenwart von Bromverbindungen

In einem 4 l Vierhalskolben mit Wasserauskreiser, Turborührer 1200 Umdrehungen pro Minute (UpM), Thermometer, und Gaseinleitungsrohr wird folgendes Gemisch unter Begasung mit 50 Nl/Std. Stickstoff zum leichten Rückfluß erhitzt.

| | | | | |
|---|---|---|---|---|
| 1000 | g p-tert.-Butylbenzoesäure | = | 5,62 Mol | 15,72 Mol |
| 1500 | g p-tert.-Butyltoluol | = | 10,1 Mol | |
| 50 | g $Co(OAc)_2 \cdot 4\ H_2O$ | = | 0,20 Mol | |

Im Wasserabscheider scheidet sich dabei ein Gemisch aus 12 g Essigsäure (0,20 Mol) und 14 g Wasser (0,78 Mol) ab. Danach liegt in der Lösung das Kobaltsalz von p-tert.-Butylbenzoesäure in Form einer blauen Lösung vor. Das Wasser-Essigsäure-Gemisch wird aus dem Wasserabscheider entnommen.

Das Reaktionsgemisch wird dann auf ca. 90°C abgekühlt und bei dieser Temperatur werden 52 g 33 %ige wäßrige Bromwasserstofflösung eingetropft = 0,21 Mol HBr. Anschließend wird das Gemisch unter ständiger Wasserabscheidung mit 100 Nl/Std. Luft begast.

Die Reaktionszeit beträgt 5 Stunden. Gegen Ende wird die Temperatur auf ca. 100°C zwecks Vervollständigung der Wasserabscheidung erhöht.

Es fallen an:

15 g Wasser im Wasserabscheider
2.540 g Reaktionsgemisch

Das Reaktionsgemisch hat folgende Zusammensetzung:

| | Gew.-% | (g) | (Mol) |
|---|---|---|---|
| p-tert.-Butyltoluol | 53,3 | 1328 | 8,96 |
| p.-tert.-Butylbenzaldehyd | 4,0 | 102 | 0,63 |
| p-tert.-Butylbenzoesäure (PTBB) | 38,6 | 980 | 5,50 |
| Co-Salz von PTBB ber. als Co(II) | 3,2 | 81 | 0,40 PTBB (0,20 CoII-Salz) |
| p-tert.-Butylbromid | 1,2 | 31 | 0,13 |
| übrige Nebenprodukte | 0,7 | 18 | |
| Summe | 100,0 | 2540 | 15,62 |

Aus dieser Analyse errechnen sich:

Umsatz p-tert.-Butyltoluol 11,3 %
Selektivität p-tert.-Butylbenzaldehyd 55,2 %, bezogen auf Umsatz p-tert.-Butyltoluol

Beim Abkühlen auf Raumtemperatur fällt der größte Teil der p-tert.-Butylbenzoesäure zusammen mit einem Teil des Co-Salzes aus.

Die Aufarbeitung geschieht folgendermaßen:

1. Abfiltrieren von p-tert.-Butylbenzoesäure
   Beim Absaugen des Reaktionsgemisches über eine Nutsche bei Raumtemperatur erhält man einen grünlichen Filterkuchen, der dreimal mit je 340 g kaltem p-tert.-Butyl-

toluol gewaschen wird. Es verbleiben 980 g eines p- tert.-Butyltoluol-feuchten Filterkuchens sowie 2720 g eines dunklen Filtrats.

Der Filterkuchen kann in Folgeansätzen wieder verwendet werden. Er enthält ca. 820 g p-tert.-Butylbenzoesäure und ca. 150 g p-tert.-Butyltoluol.

2. Alkali-Wäsche des Filtrats

Das Filtrat wird mit 1200 g 20 %iger KOH in $N_2$-Atm. bei 95°C intensiv durchgerührt. Beim Abstellen der Rührung liegen dann 2 Phasen vor. Die obere klare organische Phase (2350 g) wird von der dunklen unteren (wäßrigen) Phase (1570 g) bei 70 - 95°C abgetrennt.

Die organische Phase hat die folgende Zusammensetzung:

| | Gew.-% | (g) | (Mol) |
|---|---|---|---|
| p-tert.-Butyltoluol | 93 | 2186 | 14,77 |
| p-tert.-Butylbenzaldehyd | 4,3 | 101 | 0,62 |
| p-tert.-Butylbenzylalkohol | 2,7 | 63 | 0,38 |
| Summe | 100,0 | 2350 | 15,77 |

3. Fraktionierte Destillation der organischen Phase

Aus den 2350 g organischer Phase werden über eine 1,30 m hohe, mit Wendeln gefüllte Kolonne bei 13 mbar, Rücklauf 5:1, 2050 g p-tert.-Butyltoluol bis zu Kp. 78,5°C abdestilliert. Aus der Restmenge werden durch Destillation über eine Drehbandkolonne bei 13 mbar 98 g p-tert.-Butylbenzaldehyd, Kp 122 - 123°C gewonnen. Die auf den p-tert.-Butyltoluolumsatz bei der Reaktion bezogene isolierte Ausbeute beträgt somit 53 %.

4. Aufarbeitung der Alkaliwäsche

Die (1570 g) dunkle organische Phase wird mit 2300 g Wasser verdünnt und mit verdünnter Schwefelsäure zunächst

pH 7 bis 7,3 eingestellt. Dabei fällt das Cobaltsalz der p-tert.-Butylbenzoesäure als grauer, gut filterbarer Niederschlag aus. Dieser wird abfiltriert und getrocknet. Die Trockensubstanz wiegt 116 g und kann als Katalysator bei Folgeansätzen wiederbenutzt werden. Im Filtrat wird mit verdünnter Schwefelsäure pH 2 eingestellt. Dabei fällt p-tert.-Butylbenzoesäure als weißer Niederschlag aus. Es wird abfiltriert, ggf. umkristallisiert und getrocknet. Das Gewicht der Trockensubstanz beträgt 150 g.

Beispiel 2

Herstellung von p-tert.-Butylbenzaldehyd bei 180°C und bromfreien Kobaltkatalysator

In einem 500 ml Vierhalskolben mit Turborührer 1200 UpM, Wasserauskreiser, Thermometer und Gaseinleitungsrohr werden vorgelegt:

| | | | |
|---|---|---|---|
| 148 | g p-tert.-Butyltoluol | = | 1 Mol |
| 8,3 | g Kobalt(II)-Salz der p-tert.-Butylbenzoesäure = 0,02 Mol | | |
| 89 | g p-tert.-Butylbenzoesäure | = | 0,5 Mol |

Unter Begasung mit 10 Nl/Std. Luft wird das Gemisch auf 180°C erwärmt und 3 Stunden lang bei dieser Temperatur mit dem Turborührer bei 1200 UpM gerührt. Am Ende dieser Reaktionszeit liegen 248 g Reaktionsgemisch mit einem p-tert.-Butyltoluol-Gehalt von 53,5 Gew.-% und einem p-tert.-Butylbenzaldehyd-Gehalt von 4,0 Gew.-% vor.

Hieraus errechnet sich der p-tert.-Butyltoluol-Umsatz zu 10,4 % und die auf diesen Umsatz bezogene p-tert.-Butylbenzaldehydselektivität zu 61,6 %.

Die Aufarbeitung erfolgt wie in Beispiel 1.

Beispiel 3

Kontinuierliche Herstellung von 3-Phenoxybenzaldehyd

In einem 4 l Sechshalskolben mit Turborührer 1200 UpM, Wasserabscheider, Thermometer, Tropftrichter und Flüssigkeitsabnahme über Glastritte werden vorgelegt:

| | | |
|---|---|---|
| 1840 | g 3-Phenoxytoluol | = 10 Mol |
| 214 | g 3-Phenoxybenzoesäure | = 1 Mol |
| 97 | g Kobalt(II)-Salz der 3-Phenoxybenzoesäure | = 0,2 Mol |
| 104,3 | g Mn(II)-Salz der 3-Phenoxybenzoesäure | = 0,2 Mol |

Das Gemisch wird unter Rührung und Begasung mit 50 Nl/Std. Luft auf 200°C erwärmt. Bei dieser Temperatur wird die Reaktion zunächst 8 Stunden lang diskontinuierlich betrieben, bis der 3-Phenoxybenzaldehydgehalt im Reaktionsgemisch auf 4 - 5 Gew.-% angestiegen ist.

Dann werden pro Stunde 200 g des Reaktionsgemisches in einen Dünnschichtverdampfer geleitet, der mit einer Ölheizung von ca. 180°C beheizt wird. Dort werden pro Stunde ca. 160 g Destillat bei 10 - 15 mbar gewonnen. Zusätzlich laufen Schwersieder ca. 40 g/Std. ab, die aus einer Schmelze von Co- und Mn-Salzen in 3-Phenoxybenzoesäure bestehen. Letztere wird zum größten Teil zwecks Katalysatorrückführung in den Reaktionskolben zurückgeleitet. Ein Teil wird zur Entnahme von schwerflüchtigen Nebenprodukten - insbesondere 3-Phenoxybenzoesäure - sowie zur Regenerierung des Katalysators entnommen. Der Gehalt des Reaktionsgemisches an Katalysator + Schwersiedern wird mit dem Rückführ-/Entnahmeverhältnis unter 25 % gehalten.

Das Destillat wird kontinuierlich in eine Destillationskolonne geleitet. In deren Sumpf fallen pro Stunde ca. 12 g Produkt eines 3-Phenoxybenzaldehydgehaltes von ca. 80 % an. Das Destillat, Kp. 137 - 138°C bei 13 mbar,

148 g/Std., besteht aus 3-Phenoxytoluol, das in den Reaktionskolben zurückgeleitet wird.

Das Sumpfprodukt der ersten Kolonne wird in einer zweiten Kolonne diskontinuierlich aufgearbeitet. Pro Stunde Reaktionszeit werden dort gewonnen:

2 g 3- Phenoxytoluol
8 g 3-Phenoxybenzaldehyd
2 g Destillationsrückstand.

Dieses 3-Phenoxytoluol sowie der Destillationsrückstand, der im wesentlichen aus 3-Phenoxybenzylalkohol und 3-Phenoxybenzoesäure besteht, werden in den Reaktionskolben zurückgeleitet.

Neben den geschilderten Rückführ-Produkten werden folgende Substanzen pro Stunde frisch in den Reaktor gegeben:

13 g 3-Phenoxytoluol
8,6 g Kobalt(II)-Salz der 3-Phenoxybenzoesäure
9,2 g Mn(II)-Salz der 3-Phenoxybenzoesäure

Bei einer Produktion von 8g/Std. 3-Phenoxybenzaldehyd = 0,0404 Mol und einer Frisch-3-Phenoxytoluol-Zugabe von 13 g/Std. = 0,0707 Mol beträgt die Ausbeute 57,1 %.

Beispiel 4

Herstellung von Benzaldehyd

In einem 250 ml Vierhalskolben mit Turborührer 1200 UpM, Wasserabscheider, Thermometer, Gaseinleitungsrohr werden vorgelegt:

75 g Toluol = 0,815 Mol
6 g Kobalt(II)-Salz der Benzoesäure = 0,02 Mol

50 g Benzoesäure = 0,409 Mol

Unter Rührung und Begasung mit 10 Nl/Std. Luft wird das Gemisch zum schwachen Rückfluß bei Normaldruck (ca. 104°C) erwärmt und die Reaktion durch Zugabe von 5,2 g 33 %iger wäßriger HBr-Lösung = 0,021 Mol HBr gestartet. Danach wird bei 90 - 100°C unter ständigem Abtreiben des Reaktionswassers die Reaktion durchgeführt.
Nach 8 Stunden Reaktionszeit enthielt das Reaktionsgemisch (135 g) 6,1 Gew.-% Benzaldehyd, 2,5 % Benzylalkohol, 2 % Benzylbromid, 4,4 % Kobaltsalz der Benzoesäure, 46,3 % Toluol und 38 % Benzoesäure.

Die auf den Toluolumsatz bezogene Benzaldehydselektivität beträgt demzufolge 55,9 % bei 16,6 % Toluolumsatz.

Beispiel 5

Herstellung von Anisaldehyd

In einem wie im Beispiel 4 ausgestatteten 250 ml Vierhalskolben werden vorgelegt:

100 g p-Kresolmethylether = 0,819 Mol
7,2 g Kobalt(II)-Salz der Anissäure = 0,02 Mol
50 g Anissäure = 0,329 Mol

Das Reaktionsgemisch wird unter Rührung und Begasung mit 10 Nl/Std. Luft auf 90-100°C erwärmt. Bei dieser Temperatur wird die Reaktion durch Zugabe von 5,2 g 33 %iger wäßriger HBr-Lösung gestartet.

Nach 8 Stunden Reaktionszeit, unter ständigem Abtreiben des Reaktionswassers mit dem Luftstrom, enthielt das Reaktionsgemisch (160 g) 5,6 Gew.-% Anisaldehyd und 50,0 Gew.-% p-Kresolmethylether.

Hieraus ergibt sich ein Umsatz von 20 g = 20 % p-Kresolmethylether. Die Selektivität dieses Umsatzes zu Anisaldehyd beträgt ca. 40 %.

Patentansprüche:

1. Verfahren zur Herstellung von aromatischen Aldehyden durch Oxidation von Methylaromaten mit einem molekularen Sauerstoff enthaltenden Gas
in Fremdlösemittel-freier flüssiger Phase
in Gegenwart eines Kobaltkatalysators und gegebenenfalls von Brom und/oder Bromverbindungen
bei erhöhter Temperatur
im Einstufenverfahren,
dadurch gekennzeichnet, daß man die Oxidation unter ständiger Abführung des dabei gebildeten Wassers durchführt.

2. Verfahren nach Anspruch 1, dadurch gekennzeichnet, daß man als Methylaromaten Verbindungen der Formel

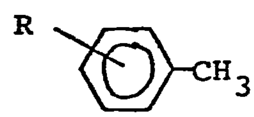 (I)

worin R = H, Halogen (vorzugsweise F, Cl, Br), tert.-Butyl, OR' (R'= Alkyl, vorzugsweise $C_1$-$C_4$-Alkyl, oder Aryl, vorzugsweise Phenyl)
verwendet.

3. Verfahren nach einem der Ansprüche 1 bis 2, dadurch gekennzeichnet, daß man als Kobaltkatalysatoren Kobaltverbindungen aromatischer Carbonsäuren - insbesondere der bei der Oxidation der eingesetzten Methylaromaten als Nebenprodukte entstehenden aromatischen Carbonsäuren - verwendet.

4. Verfahren nach einem der Ansprüche 1 bis 3, dadurch gekennzeichnet, daß man die Oxidation bei Temperaturen zwischen etwa 50 und 250°C durchführt.

5. Verfahren nach einem der Ansprüche 1 bis 4, dadurch gekennzeichnet, daß man die Oxidation bei einem Druck zwischen etwa 0,5 und 100 bar, vorzugsweise bei Normaldruck, durchführt.

6. Verfahren nach einem der Ansprüche 1 bis 5, dadurch gekennzeichnet, daß man die Oxidation bei Umsätzen nicht über etwa 25 % durchführt.

Europäisches Patentamt

# EUROPÄISCHER RECHERCHENBERICHT

0071166

Nummer der Anmeldung

EP 82 10 6563

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
| Y | CHEMICAL ABSTRACTS, Band 94, Nr. 21, Mai 1981, Seite 687, Spalte 1, Nr. 174674d, Columbus Ohio (USA); & JP - A - 80 127 327 (NIPPON SODA CO., LTD.) (02.10.1980) *Zusammenfassung* --- | 1-6 | C 07 C 45/36<br>C 07 C 47/54<br>C 07 C 47/542<br>C 07 C 47/575<br>C 07 C 47/55 |
| Y | CHEMICAL ABSTRACTS, Band 88, Nr. 23, 5. Juni 1978, Seite 556, Spalte 2, Nr. 169778n, Columbus Ohio (USA); & JP - A - 78 05 132 (MITSUBISHI CHEMICAL INDUSTRIES CO., LTD.) (18.01.1978) *Zusammenfassung* --- | 1-6 | |
| Y | GB-A- 758 665 (PETROCHEMICALS) *Seite 1; Patentanspruch 1* --- | 1 | RECHERCHIERTE SACHGEBIETE (Int. Cl. ³) |
| Y | US-A-3 139 452 (A.S.HAY) *Patentanspruch; Seite 6, Zeilen 63-66* ----- | 1 | C 07 C 45/00<br>C 07 C 47/00 |

Der vorliegende Recherchenbericht wurde für alle Patentansprüche erstellt.

| Recherchenort | Abschlußdatum der Recherche | Prüfer |
|---|---|---|
| DEN HAAG | 25-10-1982 | BONNEVALLE E.I.H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82